Europäisches Patentamt

European Patent Office

Office européen des brevets

(11) Veröffentlichungsnummer: **0 126 289**
**A2**

(12) **EUROPÄISCHE PATENTANMELDUNG**

(21) Anmeldenummer: **84104243.5**

(22) Anmeldetag: **14.04.84**

(51) Int. Cl.³: **C 12 P 21/00, C 12 N 1/00,
A 23 J 1/00**

(30) Priorität: **20.04.83 DE 3314292**

(43) Veröffentlichungstag der Anmeldung: **28.11.84
Patentblatt 84/48**

(84) Benannte Vertragsstaaten: **AT BE CH DE FR GB IT LI NL
SE**

(71) Anmelder: **HOECHST AKTIENGESELLSCHAFT,
Postfach 80 03 20, D-6230 Frankfurt am Main 80 (DE)**

(72) Erfinder: **Scharf, Udo, Dr., Altenhainer Strasse 20,
D-6233 Kelkheim (Taunus) (DE)**
Erfinder: **Schlingmann, Merten, Dr., Schneidhainer
Strasse 32a, D-6240 Königstein/Taunus (DE)**

(54) **Verfahren zur Reinigung von mikrobiellen Proteinisolaten.**

(57) Unerwünschte Geruchs- und Geschmacksstoffe können
aus mikrobiellen Proteinmaterialien entfernt werden, wenn
man diese Materialien mit wäßrigen Lösungen behandelt,
die ein Phosphat und/oder einen hydrophilen niedermolekularen Aliphaten enthalten, der mindestens zwei funktionelle Gruppen aus der Reihe Hydroxy, Formyl, Keto und
Carboxy enthält. Bevorzugte aliphatische Extraktionshilfsstoffe sind Genußsäuren, Zucker und Polyole und bei den
Phosphaten die Ortho- und Diphosphate. Die Extraktion
kann auch im Rahmen einer Nukleinsäureabreicherung entfetteter Zellmassen erfolgen.

Verfahren zur Reinigung von mikrobiellen Proteinisolaten

Aus der deutschen Offenlegungsschrift 2 137 038 ist es bekannt, Substanzen mit unangenehmem Geschmack und Geruch aus Einzeller-Proteinmaterialien, insbesondere Hefen und Bakterien, durch Extraktion mit einer wäßrigen Lösung, die 60 bis 80 Vol-% eines niederen aliphatischen Alkohols enthält, zu extrahieren.

Aus der deutschen Patentschrift 26 33 666 ist es bekannt, in mikrobiellen Zellmassen den Lipid- und Nukleinsäuregehalt dadurch zu vermindern, daß diese Zellmassen in einer ersten Stufe mit einer Extraktionsmischung aus Ammoniak und einem polaren Lösemittel aus der Reihe der niederen Alkanole, niederen Glykole und der Methyl- oder Ethylether eines niederen Glykols behandelt werden, wobei der Gesamtwassergehalt höchstens 30 Gew.-%, bezogen auf die Lösemittelmenge, beträgt und die Ammoniakkonzentration vorteilhaft 1 bis 10 Gew.-%, ebenfalls bezogen auf das Lösemittel, ist, worauf der Rückstand der Zellmassen mit Wasser behandelt wird. Hierbei werden durch die Extraktionsmischung die Lipide und ein Großteil der Geruchs- und Geschmacksstoffe extrahiert, während durch die Wasserbehandlung, die ein- oder mehrstufig erfolgen kann, die Nukleinsäuren entfernt werden. Das so erhaltene Proteinisolat ist dann weitgehend geruch- und geschmacklos und kann für viele Anwendungszwecke, auch in der menschlichen Ernährung, unmittelbar eingesetzt werden.

In der Praxis ist es jedoch nicht immer zu vermeiden, daß solchen Eiweißisolaten noch ein unangenehmer Geruch und/oder Geschmack anhaftet, der insbesondere bei speziellen Anwendungszwecken stört. Eine Extraktion mit einem wäßrigen aliphatischen Alkohol gemäß der deutschen Offenlegungsschrift 2 137 038 ist in diesen Fällen wirkungslos.

0126289

- 2 -

Es wurde nun gefunden, daß man aus mikrobiellen Proteinisolaten unerwünschte Geruchs- und Geschmacksstoffe entfernen kann, wenn man diese Isolate mit wäßrigen Lösungen
behandelt, die ein Phosphat und/oder einen hydrophilen
niedermolekularen Aliphaten enthalten, der mindestens zwei
funktionelle Gruppen aus der Reihe Hydroxy, Formyl, Keto
und Carboxy enthält. Bevorzugte Ausgestaltungen dieser
Erfindung werden im folgenden näher erläutert.

Als "niedermolekulare" Aliphaten kommen in erster Linie solche
in Betracht, die eine Kohlenstoffkette von 2 bis 6 C-Atomen enthalten bzw. im Falle von Di- und Oligosacchariden
zwei oder mehrere dieser Einheiten.

Da die Eiweißisolate oder die hieraus hergestellten funktionalisierten Derivate für die menschliche Ernährung gedacht
sind, wird man als Hilfsstoffe für die erfindungsgemäße
Extraktion wenig toxische Verbindungen heranziehen. Bevorzugt
sind deshalb physiologisch unbedenkliche Stoffe, bei den
Aliphaten also Genußsäuren, Zucker und Polyole wie Glycerin
und Zuckeralkohole. Wenn sich der Stoff aus dem Proteinisolat jedoch leicht und praktisch quantitativ abtrennen läßt,
können auch andere Hilfsstoffe, wie beispielsweise Oxalsäure,
Verwendung finden, da dann die verbleibenden Restmengen weit
unterhalb der Unbedenklichkeitsgrenze bleiben.

Als Phosphate kommen Ortho-, Oligo- und Polyphosphate in
Betracht, wobei die Polyphosphate langkettig und cyclisch
sein können. Bevorzugt sind Ortho- und Diphosphate, insbesondere in Form der leicht löslichen Natrium- und Kaliumsalze.

Die sauer reagierenden aliphatischen Verbindungen werden
vorteilhaft in Form ihrer Erdalkali- und insbesondere ihrer
Alkalisalze eingesetzt. Bevorzugt sind neben der bereits
genannten Oxalsäure die Glykolsäure, Glyoxylsäure, Brenz-

- 3 -

traubensäure, Fumarsäure, Bernsteinsäure, Weinsäure, Ascorbinsäure, insbesondere Milchsäure, Zitronensäure und Äpfelsäure, sowie Zuckersäuren aus der Aldon-, Aldar- und Uronreihe sowie die entsprechenden Lactone, insbesondere Gluconsäure-$\delta$-lacton.

Vorteilhafte Extraktionshilfsmittel sind weiterhin die Zucker, nämlich aus der Reihe der Monosaccharide die Aldosen und Ketosen der Pentose- und Hexosereihe, insbesondere Glucose und Fructose, von den Disacchariden insbessondere die Saccharose und von den Oligosacchariden insbesondere Hydrolyseprodukte der Stärke wie Stärkesirup.

Als Aliphaten, die als funktionelle Gruppen nur Hydroxygruppen haben, eignen sich neben dem bereits genannten Glycerin auch Erythrit, Pentaerythrit und vor allem Zuckeralkohole, insbesondere Sorbit.

Vorteilhaft sind auch Gemische dieser Extraktionshilfsstoffe, beispielsweise ein Phosphat in Verbindung mit einem Aliphaten wie Saccharose oder eine Mischung von aliphatischen Verbindungen, beispielsweise eine Oxalsäure enthaltende Glyoxylsäure oder ein Zuckeralkohol-Zucker-Gemisch.

Die erfindungsgemäße Reinigung kann im Anschluß an das Verfahren nach der deutschen Patentschrift 26 33 666 durchgeführt werden. Besonders vorteilhaft ist es jedoch, wenn die Extraktionshilfsmittel bei der Nukleinsäure-Extraktion zugesetzt werden. Bei einer mehrstufigen Nukleinsäure-Extraktion kann hierbei das erfindungsgemäße Extraktionshilfsmittel in der ersten, einer späteren oder in mehreren Stufen zugesetzt werden.

Die erfindungsgemäße Behandlung kann im Bereich von etwa 0 bis 100°C erfolgen, vorteilhaft bei etwa 20 bis 90°C,

- 4 -

insbesondere bei 40 bis 80°C. Bei mehrstufigen Verfahren kann die Temperatur in jeder Stufe gleich oder verschieden sein.

Je nach Einsatzzweck des Proteinmaterials können mehr oder weniger große Restmengen des Extraktionhilfsmittels im Proteinrückstand verbleiben, beispielsweise ein Zucker, wenn das Protein in süßen Lebensmitteln wie Getränken, Backwaren oder Desserts verarbeitet wird bzw. ein Phosphat, wenn das Protein in Fleisch- oder Wurstwaren, Käse oder käseähnlichen Produkten bzw. (anderen) Brotaufstrichen eingesetzt werden soll.

Der Gehalt der wäßrigen Lösungen an Phosphat und/oder Aliphat ist nach unten durch die Wirksamkeit, nach oben im Prinzip nur durch die Löslichkeit begrenzt, da ja - wie vorstehend ausgeführt - Restmengen des Extraktionsmittels in vielen Fällen im Extraktionsgut verbleiben können. Im allgemeinen wählt man Konzentrationen an Hilfsstoff von 0,1 bis 3, vorzugsweise 0,2 bis 2, insbesondere 0,5 bis 1, Gewichtsprozent.

Es ist selbstverständlich auch möglich, das erfindungsgemäße Reinigungsverfahren auf andere Eiweißstoffe anzuwenden, beispielsweise auf chemisch modifizierte mikrobielle Eiweiße.

In den folgenden Beispielen wird die Erfindung näher erläutert. Prozentangaben beziehen sich hierbei auf das Gewicht.

<u>Beispiel 1</u>

In eine Lösung von 5 g Saccharose in 1 l Wasser werden 100 g sprühgetrocknetes Proteinisolat gemäß Beispiel 1 der deutschen Patentschrift 26 33 666 unter Rühren eingetragen. Die Suspension wird auf 80°C erhitzt und bei dieser Temperatur eine halbe Stunde weitergerührt. Anschließend wird das Proteinisolat durch Zentrifugieren von der Extraktionslösung abgetrennt und getrocknet.

Das so erhaltene mikrobielle Proteinisolat zeichnet sich durch einen verbesserten Geschmack und Geruch aus. Die Zusammensetzung des Proteinanteils ist dieselbe wie vor der Extraktion.

Beispiel 2

Beispiel 1 wird mit der Abwandlung wiederholt, daß der abzentrifugierte Feststoff erneut mit 1 l Wasser verrührt wird, um anhaftende Saccharose zu entfernen. Nach erneutem Zentrifugieren wird das Proteinisolat getrocknet. Es zeigt ebenfalls einen verbesserten Geruch und Geschmack und hat die gleiche Aminosäurenzusammensetzung wie vor der Extraktion.

Beispiel 3

Ausgangsmaterial ist eine entfettete Zellmasse gemäß Beispiel 1 der deutschen Patentschrift 26 33 666, die noch die Nukleinsäuren enthält. 90 g dieser Zellmasse werden in 900 ml einer 0,5 %igen Saccharoselösung suspendiert, die Suspension auf 55°C erwärmt und bei dieser Temperatur 20 Minuten weitergerührt. Nach Abkühlen auf 30°C wird zentrifugiert. Das erhaltene Sediment wird mit 900 ml Wasser versetzt und 10 Minuten bei 20°C gerührt. Danach wird erneut zentrifugiert und das Sediment unter vermindertem Druck getrocknet.

Gegenüber einem Produkt gemäß Beispiel 1 der deutschen Patentschrift 26 33 666 zeichnet sich das Produkt durch einen verbesserten Geruch und Geschmack aus. Nukleinsäuregehalt und Aminosäurezusammensetzung sind identisch.

Beispiel 4

Beispiel 2 wird wiederholt, wobei jedoch in beiden Extraktionsstufen eine 0,5 %ige Lösung von Dinatriumorthophosphat eingesetzt wird. Man erhält ebenfalls ein Produkt von verbessertem Geruch und Geschmack und unveränderter Aminosäurezusammensetzung.

0126289

- 6 -

<u>Beispiel 5</u>

Beispiel 1 wird wiederholt, aber die Saccharose durch Sorbit ersetzt. Man erhält ein Produkt vergleichbarer Qualität.

<u>Beispiele 6 - 8</u>

Man verfährt gemäß Beispiel 1, ersetzt jedoch die Saccharose durch Milchsäure, Äpfelsäure oder Weinsäure, wobei man qualitativ vergleichbare Produkte erhält.

<u>Beispiel 9 - 11</u>

Verfährt man gemäß Beispiel 3, ersetzt jedoch die Saccharose durch Milchsäure, Stärkesirup oder Glycerin, so erhält man qualitativ vergleichbare Produkte.

<u>Beispiel 12</u>

Verfährt man gemäß Beispiel 3, setzt jedoch in beiden Extraktionsstufen eine 0,5 %ige Lösung von Natriumdiphosphat ein, so erhält man ebenfalls ein Produkt mit verbessertem Geruch und Geschmack und unveränderter Aminosäurezusammensetzung.

<u>Beispiel 13</u>

Verfährt man gemäß Beispiel 2, setzt aber in der ersten Stufe Milchsäure ein und stellt mit Natronlauge auf pH 6-7, worauf in der zweiten Stufe die Saccharoselösung angewendet wird, so erhält man ein Produkt mit verbessertem Geruch und Geschmack und unveränderter Aminosäurenzusammensetzung.

<u>Beispiel 14</u>

Verfährt man gemäß Beispiel 3, verwendet aber die in Beispiel 13 genannten Extraktionsmittel, so erhält man ebenfalls ein vergleichbares Produkt.

0126289

- 7 -

Anhang: Beispiel 1 der deutschen Patentschrift 26 33 666:

Methylomonas clara ATCC 31 226 wurde in einer Nährlösung, enthaltend Methanol als einzige Kohlenstoffquelle, Ammoniak als einzige Stickstoffquelle, Phosphat, Eisen-, Magnesium-Salze und andere übliche Spurenelemente unter aeroben Bedingungen gezüchtet. Die dabei erzeugte Bakterienzellmasse wurde von der Lösung abgetrennt und der Sprühtrocknung unterworfen.

100 g dieser Zellmasse wurden mit 300 g Methanol versetzt. Unter Rühren der Suspension wurden 10 g $NH_3$-Gas eingeleitet und gelöst. Durch Kühlen wurde die Temperatur während des Einleitens auf $25^\circ C$ - $35^\circ C$ gehalten. Das Gemisch aus Methanol, Ammoniak und Zellmasse wurde 30 Minuten bei $20^\circ C$ gerührt.

Zur Trennung der festen und flüssigen Phase wurde filtriert und der feste Rückstand einmal mit 300 ml Methanol gewaschen. Nach erneuter Filtration wurden beide Filtrate vereinigt. Diese braune Lösung enthielt die Lipide der eingesetzten Ausgangssubstanz. Methanol und Ammoniak wurden durch Vakuum-destillation (100 Torr, $40^\circ C$) entfernt. Der Rückstand, der 9,5 Gew.-% des eingesetzten Zellenmaterials betrug, war eine dunkelbraune, übelriechende Paste, die aus freien Fettsäuren, Glyceriden, Phospholipiden und Sekundärmetaboliten bestand.

Der bei der Filtration erhaltene feste Rückstand der extrahierten Zellmasse wurde im Vakuum (100 Torr) bei $40^\circ C$ 5 Stunden getrocknet. Es konnten so 90 g entfettete Zellmasse erhalten werden, die geruchlos war und eine hellere Farbe als das Ausgangsmaterial besaß.

Zur Verminderung des Nucleinsäuregehaltes wurde diese Zellmasse in 900 ml Wasser suspendiert. Der pH-Wert der durch Rühren homogenisierten Suspension betrug 6,9.

Nach Erhöhung der Temperatur auf 55°C wurde noch 20 Minuten weitergerührt, auf 30°C abgekühlt und durch Zentrifugieren in feste und flüssige Phase getrennt. Das erhaltene Sediment wurde erneut mit 900 ml Wasser vermischt und 10 Minuten bei 20°C gerührt. Danach wurde erneut zentrifugiert und das Sediment unter vermindertem Druck getrocknet.

Die Ausbeute betrug 65 g. Der Nucleinsäuregehalt war von ursprünglich 11,2 % auf 1,5 % gesunken, der Fettgehalt von 7 % auf 0,8 %. Das Produkt war in trockenem Zustand geruchlos, mit Wasser befeuchtet besaß es einen sympathischen Geruch.

Patentansprüche:

1. Verfahren zur Reinigung von mikrobiellen Proteinisolaten, dadurch gekennzeichnet, daß man diese Isolate mit wäßrigen Lösungen behandelt, die ein Phosphat und/oder einen hydrophilen niedermolekularen Aliphaten enthalten, der mindestens zwei funktionelle Gruppen aus der Reihe Hydroxy, Formyl, Keto und Carboxy enthält.

2. Verfahren nach Anspruch 1, dadurch gekennzeichnet, daß man die Isolate mit wäßrigen Lösungen von Genußsäuren, Zuckern oder Polyolen behandelt.

3. Verfahren nach Anspruch 1, dadurch gekennzeichnet, daß das Phosphat ein Natrium- oder Kaliumortho- oder -diphosphat ist.

4. Verfahren nach Anspruch 1 oder 2, dadurch gekennzeichnet, daß man sauer reagierende aliphatische Verbindungen in Form ihrer Erdalkali- oder Alkalisalze einsetzt.

5. Verfahren nach einem oder mehreren der Ansprüche 1 bis 4, dadurch gekennzeichnet, daß man als Isolate entfettete, nukleinsäurehaltige Zellmassen einsetzt.

6. Verfahren nach einem oder mehreren der vorhergehenden Ansprüche, dadurch gekennzeichnet, daß die Behandlung mehrstufig erfolgt, wobei das Extraktionsmittel gleich oder verschieden ist.

7. Verfahren nach einem oder mehreren der vorhergehenden Ansprüche, dadurch gekennzeichnet, daß die wäßrige Lösung Milchsäure, Äpfelsäure oder Zitronensäure enthält.

8. Verfahren nach einem oder mehreren der vorhergehenden Ansprüche, dadurch gekennzeichnet, daß die Behandlung in einer Stufe mit Milchsäure- und in einer folgenden Stufe mit Saccharoselösung erfolgt.

9. Verfahren nach einem oder mehreren der vorhergehenden Ansprüche, dadurch gekennzeichnet, daß die Behandlung bei etwa 0 bis 100°C, vorzugsweise 20 bis 90°C, insbesondere 40 - 80°C erfolgt.

10. Verwendung der nach Anspruch 1 bis 9 erhältlichen Produkte als Lebensmittel oder Lebensmittelzusätze.

Patentansprüche Österreich:

1. Verfahren zur Reinigung von mikrobiellen Proteinisolaten, dadurch gekennzeichnet, daß man diese Isolate mit wäßrigen Lösungen behandelt, die ein Phosphat und/oder einen hydrophilen niedermolekularen Aliphaten enthalten, der mindestens zwei funktionelle Gruppen aus der Reihe Hydroxy, Formyl, Keto und Carboxy enthält.

2. Verfahren nach Anspruch 1, dadurch gekennzeichnet, daß man die Isolate mit wäßrigen Lösungen von Genußsäuren, Zuckern oder Polyolen behandelt.

3. Verfahren nach Anspruch 1, dadurch gekennzeichnet, daß das Phosphat ein Natrium- oder Kaliumortho- oder -diphosphat ist.

4. Verfahren nach Anspruch 1 oder 2, dadurch gekennzeichnet, daß man sauer reagierende aliphatische Verbindungen in Form ihrer Erdalkali- oder Alkalisalze einsetzt.

5. Verfahren nach einem oder mehreren der Ansprüche 1 bis 4, dadurch gekennzeichnet, daß man als Isolate entfettete, nukleinsäurehaltige Zellmassen einsetzt.

6. Verfahren nach einem oder mehreren der vorhergehenden Ansprüche, dadurch gekennzeichnet, daß die Behandlung mehrstufig erfolgt, wobei das Extraktionsmittel gleich oder verschieden ist.

7. Verfahren nach einem oder mehreren der vorhergehenden Ansprüche, dadurch gekennzeichnet, daß die wäßrige Lösung Milchsäure, Äpfelsäure oder Zitronensäure enthält.

8. Verfahren nach einem oder mehreren der vorhergehenden Ansprüche, dadurch gekennzeichnet, daß die Behandlung in einer Stufe mit Milchsäure- und in einer folgenden Stufe mit Saccharoselösung erfolgt.

*Österreich*

9. Verfahren nach einem oder mehreren der vorhergehenden Ansprüche, dadurch gekennzeichnet, daß die Behandlung bei etwa 0 bis 100$^{\circ}$C, vorzugsweise 20 bis 90$^{\circ}$C, insbesondere 40 - 80$^{\circ}$C erfolgt.